# FASCICULE DE BREVET EUROPEEN

(11) **EP 0 984 902 B1**
(45) Date de publication et mention de la délivrance du brevet: **27.11.2002**
(21) Numéro de dépôt: 99909015.2
(22) Date de dépôt: 17.03.1999
(51) Int. Cl.: C01B 25/32

(54) **PROCEDE DE PREPARATION D'UN BIOMATERIAU A BASE D'HYDROXYAPATITE, BIOMATERIAU OBTENU ET APPLICATION CHIRURGICALE OU DENTAIRE**
VERFAHREN ZUR HERSTELLUNG EINES HYDROXYAPATIT ENTHALTENDES BIOMATERIALS, HERGESTELLTES BIOMATERIAL UND CHIRURGISCHE ODER ZAHNÄRTZLISCHE ANWENDUNG
METHOD FOR PREPARING A BIOMATERIAL BASED ON HYDROXYAPATITE, RESULTING BIOMATERIAL AND SURGICAL OR DENTAL USE

(30) Priorité: 20.03.1998 FR 9803459
(43) Date de publication de la demande: 15.03.2000
(73) Titulaire: Teknimed S.A., 65502 Vic en Bigorre, Cédex (FR)
(72) Inventeur: LACOUT, J., Ecole de Chimie, Pl. des Hauts Murats, F-31006 Toulouse Cedex 6 (FR); HATIM, Zinéb, Casablanca (MA); FRACHE-BOTTON, Michèle, F-31130 Fonsegrives (FR)
(74) Mandataire: Thébault, Jean-Louis
(86) Numéro de dépôt international: FR9900595
(87) Numéro de publication internationale: WO99048809

(56) Documents cités:
- EP-A- 0 323 632
- EP-A- 0 416 761
- WO-A-95/08319
- FR-A- 2 693 716

## Description

L'invention concerne un procédé de préparation d'un biomatériau essentiellement constitué d'une hydroxyapatite phosphocalcique de rapport atomique Ca/P compris entre 1,50 et 1,67. L'invention s'étend au biomatériau obtenu et à des applications de celui-ci, en particulier pour un comblement ou restauration dentaire ou osseux. L'invention vise également un set chirurgical ou dentaire en vue de la mise en oeuvre de cette application.

Les hydroxyapatites phosphocalciques sont bien connues et de plus en plus utilisées dans le domaine chirurgical ou dentaire en raison de leurs propriétés de biocompatibilité et d'ostéoconduction. Elles peuvent être utilisées en art dentaire pour le comblement parodontal, la restauration des crêtes osseuses, le comblement de kystes ou d'alvéoles après extraction dentaire... et, en chirurgie orthopédique, pour le comblement de défect osseux, le comblement interstitiel entre prothèse et os cortical, l'injection dans les corps vertébraux... Le matériau ainsi mis en place peut éventuellement contenir des substances actives qui, après durcissement in situ du matériau, sont lentement diffusées.

La préparation des hydroxyapatites phosphocalciques est essentiellement réalisée selon deux voies différentes conduisant chacune à des applications spécifiques :
- d'une part, une préparation in situ, dans laquelle le durcissement de l'hydroxyapatite est réalisé sur le site d'utilisation à basse température (en particulier in vivo à la température corporelle), cette préparation permettant la mise en oeuvre des applications chirurgicales ou dentaires précitées (cette préparation qui possède une phase de durcissement sur le site d'utilisation est désignée par la suite « préparation in situ » même si certaines phases peuvent être réalisées en dehors du site d'utilisation),
d'autre part, une préparation industrielle conduisant soit à des apatites pulvérulentes ou faiblement liées ayant des propriétés mécaniques limitées, soit à des céramiques apatitiques après traitement thermique haute température ou traitement de compression en présence d'un liant.

Les apatites industrielles ci-dessus évoquées qui bénéficient de bonnes propriétés mécaniques sont bien entendu inutilisables dans des applications où un site opératoire chirurgical ou osseux de forme quelconque doit être comblé ou restauré, puisqu'elles impliquent pour obtenir leur dureté un traitement industriel dans des conditions sévères. En outre, les apatites industrielles calcinées sont toujours stoechiométriques et présentent une faible surface spécifique (inférieure à 10 m²/g) et une faible solubilité (produit de solubilité égal à 10⁻¹¹⁹): ces propriétés rendent le matériau difficilement biorésorbable, ce qui représente un inconvénient dans la plupart des applications chirurgicales ou dentaires (où un lent remplacement par l'os du matériau implanté est généralement recherché).

La présente invention vise les apatites « à préparation in situ », c'est à dire dont le durcissement peut être effectué à basse température in situ sur le site d'utilisation. Bien entendu, ces apatites peuvent, le cas échéant, être utilisées dans d'autres applications comme le sont les apatites industrielles.

Les apatites à préparation in situ connues sont généralement préparées comme des ciments en gâchant avec de l'eau une poudre contenant un ou des phosphates calciques choisis parmi les phosphates connus : phosphate monocalcique hydraté ou non, phosphate dicalcique hydraté ou non, phosphate tricalcique α ou β, phosphate tétracalcique (brevets US 461.053, EP 0.416.761, FR 2.693.716...). Certains auteurs ont proposé de dissoudre préalablement dans l'eau de gâchage du phosphate monocalcique de façon à éviter une hydratation intempestive de ce phosphate dans la poudre (WO 95/083149). Les apatites obtenues par ces méthodes connues présentent essentiellement deux défauts majeurs. En premier lieu, leurs propriétés mécaniques sont médiocres (résistance à la compression de l'ordre de 5 à 8 Mégapascals), ce qui , dans de nombreux cas, est un inconvénient majeur (comblement parodontal, injection dans les corps vertébraux, comblement interstitiel pour le scellement de prothèse, coins d'ostéotomie durcis in situ...).
En outre, on observe très souvent avec ces produits connus la formation de « grumeaux», des temps de prise non reproductibles, et un délitement du matériau après sa mise en place in vivo en raison des liquides biologiques présents ; ces défauts conduisent soit à de graves difficultés de mise en place, soit à une qualité médiocre de l'implant obtenu (mauvais remplissage, mauvaise adhérence dans le site opératoire) , soit un transport très gênant de particules délitées hors du site opératoire. Ceci est particulièrement grave en situation chirurgicale et amène beaucoup de chirurgiens à rejeter ce type de produit après une ou des expériences malheureuses.

La présente invention se propose de fournir un procédé de préparation in situ d'un biomatériau apatitique, bénéficiant d'une bonne reproductibilité et d'une mise en oeuvre plus sûre et plus facile que les procédés connus (homogénéité du produit, temps de prise constant, facilité de mise en forme et de modelage, absence de délitement).

Un autre objectif est de permettre l'obtention d'un biomatériau présentant des propriétés mécaniques améliorées par rapport aux matériaux apatitiques connus préparés in situ.

Un autre objectif est de permettre l'obtention d'un biomatériau bénéficiant d'une solubilité et d'une surface spécifique significativement plus importantes que les biomatériaux apatitiques existants.

Un objectif de l'invention est en particulier de fournir un biomatériau apatitique qui combine d'excellentes propriétés de résistance à la compression, une bonne solubilité en milieu vivant et une surface spécifique élevée.

A cet effet, le procédé visé par l'invention en vue de préparer un biomatériau dont le durcissement s'effectue sur le site d'utilisation, en particulier à froid sur un site opératoire chirurgical ou dentaire (température inférieure à environ 40°C), est du type dans lequel on mélange des phosphates calciques pour obtenir une hydroxyapatite de rapport atomique Ca/P compris entre 1,50 et 1,67 ; le procédé de l'invention se caractérise en ce que :
a) on prépare préalablement un produit solide pulvérulent à partir d'une poudre de phosphate tricalcique et d'une poudre de phosphate tétracalcique en mélangeant lesdites poudres de façon que le rapport atomique Ca/P du produit obtenu soit sensiblement compris entre 1,40 et 1,90,
b) on prépare une solution aqueuse ou des solutions aqueuses à mélanger au produit solide pulvérulent, la ou lesdites solutions contenant des ions calcium et des ions phosphates de façon que le rapport atomique global Ca/P des solutions soit supérieur à 2,20 et que le rapport atomique Ca/P de chaque solution soit inférieur à 0,50,
c) on mélange la ou les solutions aqueuses et le produit solide pulvérulent, le cas échéant avec une addition d'eau, de façon que le rapport pondéral liquide/solide du mélange final obtenu soit compris entre 0,30 et 0,65 afin d'obtenir une pâte homogène de rapport atomique Ca/P compris entre 1,50 et 1,67, ladite pâte étant mise en place sur le site d'utilisation en vue de son durcissement in situ.

Les expérimentations ont montré que cette préparation à partir, d'une part, d'un produit solide pulvérulent contenant du phosphate tricalcique et du phosphate tétracalcique, d'autre part, d'une solution aqueuse contenant des ions calciums et des ions phosphates conduit à l'obtention d'un mélange pâteux naturellement homogène n'ayant aucune tendance à grumeler, et présentant une prise progressive et régulière et une durée de prise constante (pour une composition donnée) à la quadruple condition que :
Le rapport Ca/P du produit solide pulvérulent soit compris dans la plage sus-indiquée (avantageusement entre1,70 et 1,85),
Le rapport Ca/P de la solution ou de chaque solution si plusieurs solutions sont utilisées soit inférieur à 0,50 (avantageusement inférieur à 0,40),
Le rapport Ca/P de la solution ou le rapport global Ca/P de l'ensemble des solutions si plusieurs solutions sont utilisées soit supérieur à 0,20 (avantageusement supérieur à 0,35),
Le rapport pondéral liquide/solide du mélange réalisé est compris entre 0,30 et 0,65.

Le mélange pâteux malléable ainsi obtenu est disposé sur le site d'utilisation où il réalise sa prise et où il poursuit ensuite son durcissement. Le site d'utilisation peut être un site opératoire chirurgical ou dentaire et la prise et le durcissement s'effectuent à la température du corps (température inférieur à 40°C). Le site peut également être un moule pour réaliser une pièce, la prise et le durcissement pouvant être accomplis à froid ou à faible température (notamment inférieure à 90° C) afin d'augmenter la cinétique du durcissement. Les durées de prise peuvent varier entre 10 et 45 minutes en fonction de la composition dans les plages sus-indiquées. Le durcissement conduisant aux propriétés finales du biomatériau est obtenu au terme d'une durée de 2 à 4 jours à la température corporelle et de quelques heures à une température comprise entre 60° C et 100° C. Les analyses du biomatériau obtenu ont permis de constater que celui-ci est essentiellement constitué par une hydroxyapatite microcristalline de rapport atomique Ca/P compris entre 1,50 et 1,67, présentant en combinaison les caractéristiques suivantes :
une résistance à la compression sensiblement comprise entre 15 et 25 Mégapascals,
une solubilité correspondant à un produit de solubilité compris entre 10⁻⁹⁴ et 10⁻¹⁰⁰,
et une surface spécifique sensiblement comprise entre 20 et 120 m²/g.

Les tests de compression ont été réalisés sur des ciments mis en forme à l'aide de moules en "Plexiglas" de 6 mm de diamètre et de 6 mm de hauteur. Après la mise en forme, les moules sont placés dans de l'eau désionisée à 37° C. Après 24 heures, les ciments sont démoulés puis conservés dans les même conditions (eau à 37°C). Les tests ont été réalisés au bout de 26 jours de maturation. Avant les tests en compression les éprouvettes sont séchées. L'essai en compression est effectué en soumettant l'éprouvette cylindrique à deux forces coaxiales opposées en la plaçant entre les plateaux d'une presse. L'échantillon est soumis à une contrainte uni-axiale qui s'applique à une vitesse constante de 0,5 mm/min. Un logiciel associé permet de tracer directement la contrainte en fonction du taux de déformation. La rupture est déterminée par un brusque changement de l'allure de la courbe.

La solubilité est déterminée en mettant en suspension dans de l'eau déminéralisée et décarbonatée (100ml) une quantité déterminée de ciment (50 mg) de granulométrie comprise entre 20 et 50 microns. La suspension est agitée plusieurs fois par jours pendant 30 jours. A l'issue de cette période, on mesure le pH et on effectue un prélèvement afin de doser par ICPMS le phosphore et le calcium. On peut ensuite déterminer par calcul le produit de solubilité.

La mesure de la surface spécifique est effectué par un analyseur de type "Micromeritics Flow Sorb II 2300", sur un échantillon de ciment broyé.

Aucun matériau apatitique connu ne combine l'ensemble de ces propriétés qui s'avèrent essentielles dans de très nombreuses applications, en particulier chirurgicales ou dentaires. En outre, ce matériau présente une microporosité de l'ordre de 30 % à 55 %, ce qui est remarquable compte tenu de ses propriétés mécaniques. Cette porosité (connue en soi) permet la circulation des fluides biologiques dans les applications in vivo ce qui favorise la bio-intégration du biomatériau et sa biorésorbabilité au cours du temps.

Il semble que les avantages précités du procédé et les propriétés du biomatériau obtenu découlent de la formation intermédiaire, lors du mélange et de la prise, de grains de grosse taille constante, qui, par la suite lors de leur évolution vers l'apatite, conduisent à une morphologie à grosses aiguilles régulières et enchevêtrées. Cet enchevêtrement d'aiguilles conditionne les propriétés mécaniques, la porosité et les propriétés de surface.

En vue de faciliter le travail du praticien, en particulier dans les applications chirurgicales ou dentaires, le produit solide pulvérulent et la solution aqueuse sont préparés par doses disposées dans des conteneurs séparés telles que le rapport pondéral global liquide/solide soit compris entre 0,30 et 0,65 et que le rapport atomique global Ca/P soit compris entre 1,50 et 1,67. Il suffit au praticien de mélanger ces doses de façon homogène sans addition d'eau pour obtenir une pâte prête à l'emploi sur le site d'utilisation ; de préférence la dose liquide sera mélangée à la dose solide en réalisant un gâchage au fur et à mesure du mélange.

Le produit solide pulvérulent est avantageusement préalablement traité de sorte que le diamètre moyen des grains D₅₀ soit compris entre 15 et 20 microns et que son diamètre de coupure D₉₅ soit égal à 100 microns. On évite ainsi la présence de grains de grosses tailles qui peuvent favoriser la formation de grumeaux ; en outre, une telle répartition granulométrique serrée (grains homogènes et fins) contribue à la réalisation d'une prise progressive et régulière, parfaitement reproductible.

Selon un mode de mise en oeuvre préféré, on prépare le produit solide pulvérulent en mélangeant une poudre de phosphate tricalcique α et une poudre de phosphate tétracalcique. Par poudre de phosphate tricalcique α, on entend une poudre contenant au moins 85 % de phosphate de cette forme. Le phosphate tricalcique α est beaucoup plus soluble que la forme β et il conduit à une cinétique de prise très uniforme et plus grande et à et à l'obtention d'une pâte de meilleure homogénéité.

Par ailleurs, la solution aqueuse est de préférence préparée en mélangeant à l'eau de l'acide phosphorique avec de l'hydroxyde de calcium et/ou du carbonate de calcium. L'utilisation de ces espèces évite la présence de contre-ions dans le produit obtenu et conduit à l'obtention d'une apatite de grande pureté (à noter que, en milieu acide, le carbonate est éliminé sous forme de CO₂). L'emploi du carbonate de calcium conduit à une solution ayant un pH de l'ordre de 2,25 plus élevé que si le calcium est apporté par l'hydroxyde (pH de la solution de l'ordre de 1,75). La prise est plus lente dans le premier cas. Le cas échéant, un mélange d'hydroxyde et de carbonate peut être utilisé pour ajuster la durée de prise à une valeur souhaitée. Cette durée de prise peut également être ajustée par ajout d'un acide en faible quantité adaptée pour abaisser le pH de la solution sans toutefois atteindre une valeur égale ou inférieure à 1 (une tendance au grumelage apparaît à partir de cette valeur).

Il est possible d'ajouter au produit solide pulvérulent et/ou à la solution aqueuse d'autres additifs destinés à accroître encore la régularité de prise de la pâte. Par exemple, un glycérophosphate notamment glycérophosphate de sodium, de potassium, ou de calcium peut être ajouté de sorte que le pourcentage pondéral de ce composé rapporté au mélange final soit inférieur à 15 %. Ce composé contribue à une amélioration de la régularité de prise et de prise et diminue légèrement la vitesse de prise. Dans le cas du glycérophosphate de calcium, on tient compte de l'apport de calcium dû à l'ajout de ce composé pour préparer et doser les produits initiaux (produit solide pulvérulent, solution aqueuse) afin d'obtenir un rapport atomique final Ca/P compris dans la plage précitée. Le surplus de calcium apporté par le glycérophosphate de calcium peut en particulier être compensé par un ajout approprié d'acide phosphorique dans la solution : ceci conduit à un abaissement du pH de celle-ci et à une réduction des temps de prise qui peut être recherchée dans certaines applications.

On peut également ajouter au produit solide pulvérulent et/ou à la solution aqueuse de l'acide lactique de sorte que le pourcentage pondéral de ce composé rapporté au mélange final soit inférieur à 4 %. Ce composé possède un double effet : un effet immédiat consistant à ralentir la cristallisation du mélange et accroître la régularité de prise et à augmenter la durée de celle-ci, et un effet ultérieur lors du durcissement provenant de la formation de lactate et conduisant à un matériau de dureté accrue.

Il est également possible d'ajouter au produit solide pulvérulent et/ou à la solution aqueuse un alginate ou de la gomme de gar, ou du chitosane, de sorte que le pourcentage pondéral du composé rapporté au mélange final soit inférieur à 2 %. Ces composés augmentent la durée de prise et permettent le cas échéant d'adapter celle-ci à une valeur appropriée. En outre, ils confèrent au mélange obtenu des propriétés rhéologiques de glissement, rendant le mélange apte à circuler dans des conduits ou passages en vue notamment de son injection sur le site d'utilisation. De plus, le chitosane influence l'évolution chimique et cristallographique de la pâte obtenue en stabilisant une partie de celle-ci à un stade préapatitique : l'on obtient ainsi un bio-matériau de cristallinité très proche de celle de l'os, ayant une résorbabilité plus élevée.

Par ailleurs, la combinaison des propriétés de microporosité du matériau obtenu et de résorbabilité de celui-ci in vivo rend ledit matériau bien adapté pour être chargé d'une substance active afin d'assurer sa libération in situ, par diffusion et effet de résorption du matériau. Une telle substance active ajoutée au produit solide pulvérulent en quantité adaptée à l'effet recherché peut en particulier être constituée par un antibiotique, un antimitotique (notamment pour un matériau destiné à combler une tumeur cancéreuse), ou un facteur de croissance (notamment dans le cas d'un comblement osseux afin d'accélérer la régénération de l'os).

Le procédé de l'invention est particulièrement bien adapté pour la réalisation d'un comblement ou d'une restauration sur un site dentaire ou chirurgical. Le mélange de la solution aqueuse et du produit solide pulvérulent est réalisé à température ambiante, et la pâte obtenue est ensuite mise en place sur le site dentaire ou osseux en vue d'assurer son durcissement à 37° C. Le produit solide pulvérulent et la solution aqueuse sont alors préparés en dose comme déjà indiqué et conditionnés dans deux conteneurs fermés stérilisés. Un ou des additifs définis plus haut peuvent être ajoutés auxdites doses, en particulier du glycérophosphate de sodium, de potassium ou de calcium au produit solide pulvérulent.

Selon un mode de réalisation adapté pour les applications chirurgicales, le set chirurgical mis à la disposition du chirurgien comprend :
- (a) pour un set de poids moyen de 20 g:

- une dose de produit solide pulvérulent contenant entre 4,20et 4,50 g de phosphate tricalcique α, entre 5,50 et 5,80 g de phosphate tétracalcique, et le cas échéant entre 1,4 et 1,6 g de glycérophosphate de sodium, de potassium ou de calcium,
- une dose de solution aqueuse contenant entre 0,18 et 0,22 g d'hydroxyde de calcium et entre 1,30 et 1,50 d'acide phosphorique en solution dans 3,8 et 4,2 g d'eau.
- -(b) pour un set de poids moyen de 40 g : doses égales au double des doses précitées,
- (c) pour un set de poids moyen de 80 g : doses égales au quadruple des doses précitées (a).

Un tel set chirurgical permet en particulier au chirurgien de réaliser les interventions suivantes dans les meilleures conditions : comblement de défects osseux, réfection du plateau tibial, ostéotomie additive, scellement de prothèses, réfection de fond de cotyle, et dans la plupart des cas, greffe au moyen du matériau évitant d'effectuer une greffe autologue.

Selon un autre mode de réalisation adapté pour les applications dentaires, le set comprend :
- (a) pour un set de poids moyen de 1,5 g :
   - une dose de produit solide pulvérulent contenant entre 0,42 et 0,45 g de phosphate tricalcique α, entre 0,55 et 0,58 g de phosphate tétracalcique, et le cas échéant entre 0,14 et 0,16 g de glycérophosphate de sodium , de potassium ou de calcium,
   - une dose de solution aqueuse contenant entre 0,02 et 0,025 g d'hydroxyde de calcium et entre 0,13 et 0,15 g d'acide phosphorique en solution dans 0,3 et 0,4 g d'eau.
- (b) pour un set de poids moyen de 3 g : doses égales au double des doses précitées.

Un tel set dentaire permet en particulier au praticien de réaliser les interventions suivantes dans les meilleures conditions : comblement de poches parodontales, comblement d'alvéoles, rehaussement des crêtes...

Les exemples qui suivent illustrent le procédé de l'invention et les propriétés du biomatériau obtenu, le rapport L/S indiqué dans ces exemples correspond au rapport pondéral liquide/solide du mélange pâteux, désigné ciment, avant durcissement.

### EXEMPLE 1 : Préparation d'un ciment Ca/P. = 1,67 ; L/S = 0,45 ; NaGP=0%.

La préparation proposée correspond à une préparation pour une quantité finale de 145 g.
a) On prépare tout d'abord un mélange de poudre par pesées exactes comprenant les constituants suivants :
   Phosphate tétracalcique = 62,38 g (le phosphate tétracalcique est préalablement broyé de façon à ce que sa granulométrie soit inférieure à 70 microns).
   Phosphates tricalciques α = 37,62g.
   Le rapport atomique calcium/phosphate de ce mélange est égal à 1,79.
   Ce mélange est soigneusement homogénéisé au moyen d'un mélangeur de poudre.
   On prépare ensuite une solution de la façon suivante :
b) On mesure 6,1 g d'acide phosphorique concentré (d= 1,69); on ajoute lentement 1,46 g d'hydroxyde de calcium. On complète ensuite à 45 ml avec de l'eau distillée. On obtient ainsi une solution limpide, stable de rapport atomique calcium/phosphore Ca/P = 0,377.
c) On verse dans un petit mortier le mélange de poudre. On y ajoute ensuite la totalité de la solution en malaxant énergiquement au moyen d'un pilon ou d'une spatule. Le mélange est initialement granuleux mais devient très vite lisse et homogène. Après environ une à deux minutes de malaxage le mélange est laissé à reposer. Après environ 10 minutes il présente une consistance telle qu'il peut être moulé et mis en place. Il termine sa prise après environ 15 minutes.

Le ciment préparé selon cette méthode présente un rapport atomique global CA/P = 1,67 et un rapport liquide/solide L/S = 0,45.

Si l'on veut examiner le devenir de ce ciment in vitro, il est nécessaire de poursuivre le durcissement du ciment dans un milieu humide (analogue à celui rencontré en milieu biologique).

L'évolution du ciment d'un point de vue cristallographique, peut être suivie par diffraction des rayons X. On observe la disparition progressive des phases initiales du mélange et la formation après environ 72 heures d'une phase apatitique moyennement cristallisée ; cette phase est analogue à celle de la partie minérale de l'os.

On peut tout au long de l'évolution de mélange mesurer les modifications de la plasticité en utilisant un pénétromètre: celui-ci mesure la résistance à la pénétration à la surface du ciment d'une pointe de 1mm². La variation de cette résistance en fonction du temps est fournie par une courbe telle que la courbe reportée sur la figure 1. On considère qu'une valeur voisine de 250 g/mm², correspond à la limite pour mettre le ciment en forme sur le site d'utilisation. A la valeur correspondant à 300 g/mm², le ciment a perdu toute malléabilité : il a totalement fait prise. Bien entendu, ensuite, il continue son durcissement.

Dans le cas du ciment ici décrit, la valeur limite (250 g/mm²) est atteinte après 15 minutes. La résistance à la compression est de 15 MPa. Sa porosité est de 41 %.

### EXEMPLE 2 : Préparation d'un ciment Ca/P = 1,546 ; L/S = 0,45 ; NaGP = 0 %

La préparation proposée correspond à une préparation pour une quantité finale de 145 g.
a) On prépare tout d'abord un mélange de poudre réalisé par pesées exactes comprenant les constituants suivants :
   Phosphates tétracalcique = 16,8 g. Le phosphate tétracalcique est broyé de façon à ce que sa granulométrie soit inférieure à 70 microns.
   Phosphates tricalcique α= 83.3 g, tel que le rapport atomique calcium/phosphore de ce mélange soit égal à 1,573.
   Ce mélange est soigneusement homogénéisé au moyen d'un mélangeur de poudre.
   On prépare ensuite une solution de la façon suivante :
b) On mesure 1,67 g (1 ml) d'acide phosphorique concentrée (d = 1,69); on ajoute lentement 0,40 g d'hydroxyde de calcium. On complète ensuite à 45 ml avec de l'eau distillée. On obtient ainsi une solution limpide, stable de rapport atomique calcium/phosphore Ca/P = 0,378.
c) On verse dans un petit mortier le mélange de poudre. On y ajoute ensuite la totalité de la solution en malaxant énergiquement au moyen d'un pilon ou d'une spatule. Le mélange est initialement granuleux mais devient très vite lisse et homogène. Après environ une à deux minutes de malaxage le mélange est laissé à reposer. Après environ 25 minutes il a une consistance telle qu'il peut être moulé mis en place. Il termine sa prise après environ 35 minutes.

Le ciment préparé selon cette méthode présente un rapport atomique global Ca/P = 1,546 et un rapport liquide/solide L/S = 0,45.

Dans le cas de ce ciment, la valeur limite (250g/mm²) est atteinte après 30 minutes. La résistance à la compression est de 8 Mpa. Sa porosité est de 45%.

### EXEMPLE 3 : Ca/P = 1,63 ; L/S = 0,42 ; NaGP = 4,5%

Le ciment suivant est préparé comme les précédents. La composition des différentes parties est :

### Phase solide pulvérulente :

Phosphate tétracalcique = 16,8 g.

Phosphate tricalcique α = 83,3 g.

Glycérophosphate de sodium (NaGP) = 65 g tel que le rapport atomique de calcium/phosphore de ce mélange soit égal à 1,76.

### Phase liquide :

Acide phosphorique (d = 1,69) = 2,47 g.

Hydroxyde de calcium = 1,50 g.

Complétée à 43 cm³ avec de l'eau distillée.

Le rapport atomique du liquide est 0,367.

Après ce mélange, la pâte fait prise en 15 minutes. Sa dureté finale est de 20 Mpa.

### EXEMPLE 4 : Ca/P = 1,67 ; L/S = 0,43 ; NaGP = 9%

Le ciment suivant est préparé comme les précédents. La composition des différentes parties est :

### Phase solide pulvérulente :

Phosphate tétracalcique = 49,0 g.

Phosphate tricalcique α = 37,8 g.

Glycérophosphate de sodium (NaGP) = 130 g tel que le rapport atomique calcium/phosphore de ce mélange soit égal à 1,76.

### Phase liquide :

Acide phosphorique (d = 1,69 ) = 1,0 g

Hydroxyde de calcium = 1,44 g complété à 43 cm³ avec de l'eau distillée.

Le rapport atomique du liquide est 0,367.

Après mélange, la pâte fait prise en 15 minutes. Sa dureté finale est de 20 Mpa. Sa porosité est 40%.

### EXEMPLE 5 : Ca/P = 1,58 ; L/S = 0,45 ; NaGP = 9% Ca/P = 1,546 ; L/S 0,45 ; NaGP = 0%.

Le ciment suivant est préparé comme les précédents. La composition des différentes parties est :

### Phase solide pulvérulente :

Phosphate tétracalcique = 49,0g

Phosphate tricalcique α = 62,0g.

Glycérophosphate de sodium (NaGP) = 13 g tel que le rapport calcium/Phosphore de ce mélange soit égal à 1,727.

### Phase liquide :

Acide phosphorique (d = 1,99) = 0,6ml.

Hydroxyde de calcium = 1,44 g complété à 43 ml avec de l'eau distillée.

Le rapport atomique du liquide est 0,377.

Après mélange, la pâte fait prise en 20 minutes. Sa dureté finale est de 10 Mpa. Sa porosité est 48%.

### EXEMPLE 6 : industriellement on prépare de la façon suivante des quantités de 2 kg de poudre et de 1000 cm³ de solution contenant respectivement :

### Produit solide pulvérulent :

Phosphate trétracalcique = 980 g.

Phosphate tricalcique α = 758,0 g

Glycérophosphate = 260 g.

### Solution :

Hydroxyde de calcium = 34,0 g.

Acide phosphorique (d = 1,69) = 138 g.

On complète à 1000 cm³.

A partir de ces produits on prépare des sets chirurgicaux contenant:

11,6 g de solide pulvérulents mis en flacon serti et 5 cm³ (soit 5,8 g) de solution mise en ampoule scellée. Le tout est placé dans une boite en carton, référencé et stérilisé par rayonnement ionisant.

A partir de ces produits on prépare des sets dentaires contenant :
1,0 g de solide pulvérulent mis en flacon sertie et 0,5 cm³ de solution en ampoule scellée. Le tout est placé dans une boite en carton, référencé et stérilisé par rayonnement ionisant.

### EXEMPLE 7 : Préparation d'un ciment contenant du chitosane, tel que : Ca/P = 1,63 ; L/S = 0,43 ; NaGP = 9% ; Chitosane = 0,5%

Le ciment est préparé comme précédemment ; les compositions des différentes parties sont les suivantes :
- Solide: Phosphate tétracalcique : 49,0 g
Phosphate tricalcique alpha : 37,9 g
Glycérophospate de sodium : 13g
- Solution: Hydroxyde de calcium : 3,4 g
Acide phosphorique (d : 1,69) = 13,8
Chistosane (Désacétylé à 50%) = 1 g

On complète à 100 ml

11,6 g de solide pulvérulent sont mélangés avec 5 ml de la solution. Le tout est malaxé pendant 1 minute. Après une durée de 15 minutes, le ciment présente une dureté suffisante pour être placé en site chirurgical ; cependant, sa prise n'est en fait complète qu'après 1 heure.

Lorsque le durcissement est effectué en conditions humides analogues à celles d'un milieu biologique le ciment après une journée totale d'évolution possède une résistance à l'écrasement égal à 20 Mpa.

La diffraction des rayons X effectuée après 24 heures et après une semaine montre que l'évolution vers la phase apatitique n'est pas totale. Une partie du ciment a évolué vers une phase préapatitique du type phosphate octocalcique.

## Revendications

1. Procédé de préparation d'un biomatériau comportant une phase de durcissement sur un site d'utilisation, du type dans lequel on mélange des phosphates calciques pour obtenir une hydroxyapatite de rapport atomique Ca/P compris entre 1,50 et 1,67, le dit procédé étant **caractérisé en ce que**:
a) on prépare préalablement un produit solide pulvérulent à partir d'une poudre de phosphate tricalcique et d'une poudre de phosphate tétracalcique en mélangeant lesdites poudres de façon que le rapport atomique Ca/P du produit pulvérulent obtenu soit sensiblement compris entre 1,40 et 1,90,
b) on prépare une solution aqueuse ou des solutions aqueuses à mélanger au produit solide pulvérulent, la ou lesdites solutions contenant des ions calcium et des ions phosphate de façon que le rapport atomique global Ca/P des solutions soit supérieur à 0,20 et que le rapport atomique Ca/P de chaque solution soit inférieur à 0,50,
c) on mélange la ou les solutions aqueuses et le produit solide pulvérulent, le cas échéant avec une addition d'eau, de façon que le rapport pondéral liquide/solide du mélange final obtenu soit compris entre 0,30 et 0,65 afin d'obtenir une pâte homogène de rapport atomique Ca/P compris entre 1,50 et 1,67, ladite pâte étant mise en place sur le site d'utilisation en vue de son durcissement in situ.

2. Procédé de préparation d'un biomatériau comportant une phase de durcissement sur un site d'utilisation, du type dans lequel on mélange des phosphates calciques pour obtenir une hydroxyapatite de rapport atomique Ca/P compris entre 1,50 et 1,67, le dit procédé étant **caractérisé en ce que** :
a) on prépare préalablement un produit solide pulvérulent à partir d'une poudre de phosphate tricalcique et d'une poudre de phosphate tétracalcique en mélangeant lesdites poudres de façon que le rapport atomique Ca/P du produit pulvérulent obtenu soit sensiblement compris entre 1,40 et 1,90,
b) on prépare une dose d'une solution aqueuse contenant à la fois des ions calcium et des ions phosphate, lesdites doses de produit solide et de solution aqueuse étant telles que le rapport pondéral global liquide/solide soit compris entre 0,30 et 0,65 et que le rapport atomique global Ca/P soit compris entre 1,50 et 1,67, et
c) on mélange de façon homogène la dose de produit solide pulvérulent et la dose de solution aqueuse sans addition d'eau.

3. Procédé selon l'une des revendications 1 ou 2, dans lequel on prépare le produit solide pulvérulent à partir de poudre de phosphate tricalcique et de poudre de phosphate tétracalcique de sorte que le diamètre moyen des grains D₅₀ dudit produit solide pulvérulent soit compris entre 15 et 50 microns et que son diamètre de coupure D₉₅ soit égal à 100 microns.

4. Procédé selon l'une des revendications 1, 2 ou 3, dans lequel on prépare le produit solide pulvérulent en mélangeant une poudre de phosphate tricalcique α et une poudre de phosphate tétracalcique.

5. Procédé selon l'une des revendications 1, 2, 3 ou 4, dans lequel on prépare la solution aqueuse en mélangeant dans de l'eau de l'acide phosphorique avec de l'hydroxyde de calcium et/ou du carbonate de calcium.

6. Procédé selon l'une des revendications 1 à 5, dans lequel on abaisse le pH de la solution aqueuse obtenue par ajout d'un acide en faible quantité adaptée pour le pH de la solution demeure supérieur à 1.

7. Procédé selon l'une des revendications 1 à 6, **caractérisé en ce que** (a) la produit solide pulvérulent est préparé de façon que son rapport atomique Ca/P soit compris entre 1,70 et 1,85, (b) la solution aqueuse est préparée de façon à contenir à la fois des ions calcium et des ions phosphate dans un rapport atomique Ca/P sensiblement compris entre 0,35 et 0,40.

8. Procédé selon l'une des revendications 1 à 7, dans lequel on ajoute au produit solide pulvérulent et/ou à la solution aqueuse un glycérophosphate, notamment glycérophosphate de sodium, de potassium, ou de calcium, de sorte que le pourcentage pondéral de ce composé rapporté au mélange final soit inférieur à 15%.

9. Procédé selon l'une des revendications 1 à 10, **caractérisé en ce qu'**on ajoute au produit solide pulvérulent et/ou à la solution aqueuse de l'acide lactique de sorte que le pourcentage pondéral de ce composé rapporté au mélange final soit inférieur à 4%.

10. Procédé selon l'une des revendications 1 à 9, **caractérisé en ce qu'**on ajoute au produit solide pulvérulent et/ou à la solution aqueuse un alginate ou de la gomme de gar ou du chitosane, de sorte que le pourcentage pondéral dudit composé rapporté au mélange final soit inférieur à 2%.

11. Procédé selon l'une des revendications 1 à 10, dans lequel on ajoute au produit solide pulvérulent une substance active, en particulier antibiotique, antimitotique ou facteur de croissance.

12. Procédé selon l'une des revendications 1 à 11, en vue d'une application dentaire ou osseuse, dans lequel le mélange de la solution aqueuse et du produit solide pulvérulent est réalisé à température ambiante, le dit mélange étant ensuite amené à durcir à une température inférieure à 40° C.

13. Procédé selon l'une des revendications 1 à 11, en vue de fabriquer par moulage une pièce à usage chirurgical ou prothétique, dans lequel :
. le mélange de la solution aqueuse et du produit solide pulvérulent est réalisé à température ambiante,
. ledit mélange est ensuite disposé dans un moule et laissé dans celui-ci à température ambiante jusqu'à l'obtention d'une prise suffisante pour permettre le démoulage,
. la pièce est alors démoulée et chauffée à une température comprise entre 50°C et 90°C dans une atmosphère humide ou aqueuse jusqu'à obtenir son durcissement complet.

14. Biomatériau essentiellement constitué par une hydroxyapatite microcristalline de rapport atomique Ca/P compris entre 1,50 et 1,67, obtenu par le procédé selon l'une quelconque des revendications 1 à 13, présentant les caractéristiques suivantes :
. une résistance à la compression sensiblement comprise entre 15 et 25 Mégapascals,
. une solubilité correspondant à un produit de solubilité compris entre 10⁻⁹⁴ et 10⁻¹⁰⁰,
. et une surface spécifique sensiblement comprise entre 20 et 120 m²/g.

15. Set chirurgical ou dentaire pour la mise en oeuvre du procédé selon l'une quelconque des revendications 1 à 13, **caractérisé en ce qu'**il comprend:
. une dose de produit solide pulvérulent comportant un mélange de phosphate tricalcique et de phosphate tétracalcique dans un rapport atomique Ca/P sensiblement compris entre 1,40 et 1,90, ladite dose étant conditionnée dans un premier conteneur fermé stérilisé,
. une dose de solution aqueuse contenant des ions calcium et des ions phosphate dans un rapport atomique Ca/P sensiblement compris entre 0,20 et 0,50 ladite dose étant conditionnée dans un second conteneur fermé stérilisé,
- lesdites doses de produit solide pulvérulent et de solution aqueuse étant telles que le rapport pondéral global liquide/solide soit compris entre 0,30 et 0,65 et que le rapport atomique global Ca/P soit compris entre 1,50 et 1,67

16. Set chirurgical ou dentaire selon la revendication 15, dans lequel la dose de produit solide pulvérulent contient un pourcentage pondéral de glycérophosphate de sodium, de potassium ou calcium compris entre 5% et 15%.

17. Set chirurgical selon l'une des revendications 15 ou 16, **caractérisé en ce qu'**il comprend des doses correspondant à une, deux ou quatre fois les quantités unitaires suivantes :
produit solide pulvérulent contenant entre 4,2 et 4,5 g de phosphate tricalcique α et entre 5,5 et 5,8 g de phosphate tétracalcique, et le cas échéant entre 1,4 et 1,6 g de glycérophosphate de sodium, de potassium, ou de calcium,
. solution aqueuse contenant entre 0,18 et 0,22 g d'hydroxyde de calcium et entre 1,3 et 1,5 g d'acide phosphorique, en solution dans 3,8 et 4,2 g d'eau.

18. Set dentaire selon l'une des revendications 15 ou 16, **caractérisé en ce qu'**il comprend des doses correspondant à une ou deux fois les quantités unitaires suivantes :
. produit solide pulvérulent contenant entre 0,42 et 0,45 g de phosphate tricalcique α et entre 0,55 et 0,58 g de phosphate tétracalcique, et le cas échéant entre 0,14 et 0,16 g de glycérophosphate de sodium, de potassium, ou de calcium,
. solution aqueuse contenant entre 0,02 et 0,025 g d'hydroxyde de calcium et entre 0,13 et 0,15 g d'acide phosphorique, en solution dans 0,3 et 0,4 g d'eau.

## Patentansprüche

1. Verfahren zur Herstellung eines Biomaterials, mit einem Aushärtungsstadium auf einem Verwendungsort und von dem Typ, bei dem man Calciumphosphate mischt, um ein Hydroxyapatit mit einem atomaren Verhältnis Ca/P zwischen 1,50 und 1,67 zu erhalten, wobei das Verfahren **dadurch gekennzeichnet ist, daß**:
a) man zunächst ein festes, pulvriges Produkt ausgehend von einem Tricalciumphosphatpulver und einem Tetracalciumphosphatpulver durch Mischen der Pulver derart, daß das atomare Verhältnis Ca/P des erhaltenen pulvrigen Produkts im wesentlichen zwischen 1,40 und 1,90 beträgt, herstellt,
b) man eine wässrige Lösung oder wässrige Lösungen zum Mischen mit dem festen pulvrigen Produkt herstellt, wobei die Lösung oder die Lösungen Calciumionen und Phosphationen derart enthalten, daß das atomare Gesamtverhältnis Ca/P der Lösungen größer als 0,20 und das atomare Verhältnis Ca/P jeder Lösung kleiner als 0,50 ist,
c) man die wässrige Lösung oder die wässrigen Lösungen und das feste pulvrige Produkt gegebenenfalls unter Zusatz von Wasser mischt, so daß das Gewichtsverhältnis flüssig/fest der erhaltenen Endmischung zwischen 0,30 und 0,65 beträgt, um eine homogene Paste zu erhalten, deren atomares Verhältnis Ca/P zwischen 1,50 und 1,67 beträgt, wobei die Paste angesichts ihrer Aushärtung in situ auf den Verwendungsort aufgetragen wird.

2. Verfahren zur Herstellung eines Biomaterials mit einem Aushärtungsstadium auf einem Verwendungsort und von dem Typ, bei dem man Calciumphosphate mischt, um ein Hydroxyapatit mit einem atomaren Verhältnis Ca/P zwischen 1,50 und 1,67 zu erhalten, wobei das Verfahren **dadurch gekennzeichnet ist, daß**:
a) man zunächst ein festes, pulvriges Produkt ausgehend von einem Tricalciumphosphatpulver und einem Tetracalciumphosphatpulver durch Mischen der Pulver derart daß das atomare Verhältnis Ca/P des erhaltenen pulvrigen Produkts im wesentlichen zwischen 1,40 und 1,90 liegt, herstellt,
b) man eine Dosis einer wässrigen Lösung herstellt, die zugleich Calciumionen und Phosphationen enthält, wobei die Dosen des festen Produkts und der wässrigen Lösung solche sind, daß das Gesamtgewichtsverhältnis flüssig/fest zwischen 0,30 und 0,65 und das atomare Gesamtverhältnis Ca/P zwischen 1,50 und 1,67 liegt, und
c) man die Dosis des festen pulvrigen Produkts und die Dosis der wässrigen Lösung ohne Zusatz von Wasser homogen mischt.

3. Verfahren nach Anspruch 1 oder 2, bei dem man das feste pulvrige Produkt ausgehend von Tricalciumphosphatpulver und Tetracalciumphosphatpulver derart herstellt, daß der mittlere Durchmesser der Körnchen D₅₀ des festen pulvrigen Produkts zwischen 15 und 50 µm beträgt und sein Grenzdurchmesser D₉₅ gleich 100 µm ist.

4. Verfahren nach einem der Ansprüche 1, 2 oder 3, bei dem man das feste pulvrige Produkt durch Mischen eines α-Tricalciumphosphatpulvers und eines Tetracalciumphosphatpulvers herstellt.

5. Verfahren nach einem der Ansprüche 1, 2, 3 oder 4, bei dem man die wässrige Lösung durch Mischen in Phosphorsäurelösung mit Calciumhydroxyd und/oder Calciumkarbonat herstellt.

6. Verfahren nach einem der Ansprüche 1 bis 5, bei dem man den pH der erhaltenen wässrigen Lösung durch Zusatz einer kleinen Menge einer Säure senkt, die so angepaßt ist, daß der pH der Lösung größer als 1 bleibt.

7. Verfahren nach einem der Ansprüche 1 bis 6, **dadurch gekennzeichnet, daß** (a) das feste pulvrige Produkt derart hergestellt wird, daß sein atomares Verhältnis Ca/P zwischen 1,70 und 1,85 beträgt, (b) die wässrige Lösung derart hergestellt wird, daß sie zugleich Calciumionen und Phosphationen in einem atomaren Verhältnis Ca/P im wesentlichen zwischen 0,35 und 0,40 enthält.

8. Verfahren nach einem der Ansprüche 1 bis 7, bei dem man dem festen pulvrigen Produkt und/oder der wässrigen Lösung ein Glycerophosphat zugibt, insbesondere Natrium-, Kalium- oder Calciumglycerophosphat, derart, daß das prozentuale Gewicht dieser Verbindung im Verhältnis zur endgültigen Mischung weniger als 15% beträgt.

9. Verfahren nach einem der Ansprüche 1 bis 10, **dadurch gekennzeichnet, daß** man dem festen pulvrigen Produkt und/oder der wässrigen Lösung Milchsäure derart zusetzt, daß das prozentuale Gewicht dieser Verbindung im Verhältnis zur endgültigen Mischung weniger als 4% beträgt.

10. Verfahren nach einem der Ansprüche 1 bis 9, **dadurch gekennzeichnet, daß** man dem festen pulvrigen Produkt und/oder der wässrigen Lösung ein Alginat oder Guargummi oder chitosan derart zugibt, daß das prozentuale Gewicht dieser Verbindung im Verhältnis zur endgültigen Mischung weniger als 2% beträgt.

11. Verfahren nach einem der Ansprüche 1 bis 10, bei dem man dem festen pulvrigen Produkt eine aktive, insbesondere antibiotische, antimitotische oder wachstumshemmende Substanz zufügt.

12. Verfahren nach einem der Ansprüche 1 bis 11, im Hinblick auf eine dentale oder ossale Verwendung, bei dem die Mischung der wässrigen Lösung und des festen pulvriges Produkts bei Zimmertemperatur hergestellt wird, wobei die Mischung anschließend bei einer Temperatur kleiner als 40° C ausgehärtet wird.

13. Verfahren nach einem der Ansprüche 1 bis 11, im Hinblick auf eine Abgußherstellung eines chirurgisch oder prothetisch zu verwendenden Teils, bei dem:
- die Mischung der wässrigen Lösung und des festen pulvrigen Produkts bei Zimmertemperatur hergestellt wird,
- die Mischung anschließend in eine Form gebracht und in dieser bei Zimmertemperatur gelassen wird, bis zum Erhalt einer ausreichenden Abbindung, die ein Herausnehmen aus der Form erlaubt,
- das Teil dann aus der Form entfernt und auf eine Temperatur zwischen 50 und 90° C in einer feuchten oder wässrigen Atmosphäre erhitzt wird, bis man seine vollständige Aushärtung erhält.

14. Biomaterial, das im wesentlichen aus einem mikrokristallinen Hydroxyapatit mit einem atomaren Verhältnis Ca/P zwischen 1,50 und 1,67 besteht, nach einem Verfahren gemäß einem der Ansprüche 1 bis 13 hergestellt ist und die nachfolgenden Merkmale aufweist:
- einen Druckwiderstand im wesentlichen zwischen 15 und 25 Megapascal,
- eine einem Löslichkeitsprodukt zwischen 10⁻⁹⁴ und 10⁻¹⁰⁰ entsprechende Löslichkeit,
- und eine spezifische Oberfläche im wesentlichen zwischen 20 und 120 m²/g.

15. Chirurgisches oder Dentalset zum Ausführen des Verfahrens nach einem der Ansprüche 1 bis 13, **dadurch gekennzeichnet, daß** es umfaßt:
- eine Dosis des festen pulvrigen Produkts mit einer Mischung aus Tricalciumphosphat und Tetracalciumphosphat in einem atomaren Verhältnis Ca/P im wesentlichen zwischen 1,40 und 1,90, wobei die Dosis in einem geschlossenen sterilisierten Container abgepackt ist,
- eine Dosis wässriger Lösung mit Calciumionen und Phosphationen in einem atomaren Verhältnis Ca/P im wesentlichen zwischen 0,20 und 0,50, wobei diese Dosis in einem zweiten geschlossenen sterilisierten Container abgepackt ist,
- wobei die Dosen des festen pulvrigen Produkts und der wässrigen Lösung solche sind, daß das Gsamtgewichtsverhältnis flüssig/fest zwischen 0,30 und 0,65 und atomare Gesamtverhältnis Ca/P zwischen 1,50 und 1,67 beträgt.

16. Chirurgisches oder Dentalset nach Anspruch 15, bei dem die Dosis des festen pulvrigen Produkts Natrium-, Kalium- oder Calciumglycerophosphat zwischen 5 und 15 Gew.-% enthält.

17. Chirurgisches Set nach Anspruch 15 oder 16, **dadurch gekennzeichnet, daß** es Dosen enthält, die dem Ein-, Zwei- oder Vierfachen der folgenden Einheitsmengen entsprechen:
- festes pulvriges Produkt mit α-Tricalciumphosphat zwischen 4,2 und 4,5 g und Tetracalciumphosphat zwischen 5,5 und 5,8 g, und gegebenenfalls Natrium-, Kalium- oder Calciumglycophosphat zwischen 1,4 und 1,6 g,
- wässrige Lösung mit Calicumhydroxyd zwischen 0,18 und 0,22 g und Phosphorsäure zwischen 1,3 und 1,5 g, in Lösung in 3,8 bis 4,2 g Wasser.

18. Dentalset nach Anspruch 15 oder 16, **dadurch gekennzeichnet, daß** es das Ein- oder Zweifache der folgenden Einheitsdosen enthält:
- festes pulvriges Produkt mit α-Tricalciumphosphat zwischen 0,42 und 0,45 g und Tetracalciumphosphat zwischen 0,55 und 0,58 g und gegebenenfalls Natrium-, Kalium- oder Calciumglycerophosphat zwischen 0,14 und 0,16 g,
- wässrige Lösung mit Calciumhydroxyd zwischen 0,02 und 0,025 g und Phosphorsäure zwischen 0,13 und 0,15 g, in Lösung in 0,3 bis 0,4 g Wasser.

## Claims

1. A method of preparing a biomaterial comprising a phase of hardening at a site of use, of the type wherein calcium phosphates are mixed to obtain a hydroxyapatite with a Ca/P atomic ratio from 1.50 to 1.67, **characterised in that**
a) a solid, powdery material is prepared at a preliminary stage from a tricalcium phosphate powder and a tetracalcium phosphate powder, by mixing said powders so that the Ca/P atomic ratio of the powdery material obtained is substantially from 1.40 to 1.90,
b) an aqueous solution or aqueous solutions are prepared for mixing with the solid powdery material, said solution or solutions containing calcium ions and phosphate ions so that the total Ca/P atomic ratio of the solutions is over 0.20 and the Ca/P atomic ratio of each solution is below 0.50,
c) the aqueous solution or solutions and the solid, powdery material are mixed, if appropriate with water added, so that the liquid/solid weight ratio of the final mixture obtained is from 0.30 to 0.65 in order to obtain a homogeneous paste with a Ca/P atomic ratio from 1.50 to 1.67, said paste being positioned at the site of use in order to harden *in situ.*

2. A method of preparing a biomaterial comprising a phase of hardening at a site of use, of the type wherein calcium phosphates are mixed to obtain a hydroxyapatite with a Ca/P atomic ratio between 1.50 and 1.67, **characterised in that**
a) a solid, powdery material is prepared at a preliminary stage from a tricalcium phosphate powder and a tetracalcium phosphate powder, by mixing said powders so that the Ca/P atomic ratio of the powdery material obtained is substantially from 1.40 to 1.90,
b) a measured quantity of an aqueous solution containing both calcium ions and phosphate ions is prepared, said quantities of solid material and aqueous solution being such that the total liquid/solid weight ratio is from 0.30 to 0.65 and the total Ca/P atomic ratio is from 1.50 to 1.67, and
c) the measured quantity of solid, powdery material and the measured quantity of aqueous solution are mixed without adding water.

3. A method according to claim 1 or 2, wherein the solid powdery material is prepared from tricalcium phosphate powder and tetracalcium phosphate powder, so that the mean particle diameter D₅₀ of the solid powdery material is from 15 to 50 microns and its sectional diameter D₉₅ equals 100 microns.

4. A method according to any of claims 1, 2 or 3, wherein the solid powdery material is prepared by mixing a α tricalcium phosphate powder and a tetracalcium phosphate powder.

5. A method according to any of claims 1, 2, 3 or 4, wherein the aqueous solution is prepared by mixing phosphoric acid with calcium hydroxide and/or calcium carbonate in water.

6. A method according to any of claims 1 to 5, wherein the pH of the aqueous solution obtained is lowered by adding an acid in a small quantity, adapted to keep the pH of the solution above 1.

7. A method according to any of claims 1 to 6, **characterised in that** (a) the solid, powdery material is prepared so that its Ca/P atomic ratio is from 1.70 to 1.85, and (b) the aqueous solution is prepared so as to contain both calcium ions and phosphate ions in a Ca/P atomic ratio substantially from 0.35 to 0.40.

8. A method according to any of claims 1 to 7, wherein a glycerophosphate, particularly sodium, potassium or calcium glycerophosphate, is added to the solid powdery material and/or to the aqueous solution, so that the percentage of that compound by weight relative to the final mixture is less than 15%.

9. A method according to any of claims 1 to 10, **characterised in that** lactic acid is added to the solid powdery material and/or to the aqueous solution, so that the percentage of that compound by weight relative to the final mixture is less than 4%.

10. A method according to any of claims 1 to 9, **characterised in that** an alginate or gar gum or chitosan is added to the solid powdery material and/or to the aqueous solution, so that the percentage of said compound by weight relative to the final mixture is less than 2%.

11. A method according to any of claims 1 to 10, wherein an active substance, particularly an antibiotic, antimitotic or growth factor, is added to the solid powdery material.

12. A method according to any of claims 1 to 11, for an application involving teeth or bones, wherein the aqueous solution and solid powdery material are mixed at room temperature, said mixture then being hardened at a temperature below 40°C.

13. A method according to any of claims 1 to 11 for moulding a piece for surgical or prosthetic use, wherein:
- the aqueous solution and solid, powdery material are mixed at room termperature,
- said mixture is then placed in a mould and left therein at room temperature until sufficient solidification is obtained to allow demoulding, and
- the piece is then demoulded and heated to a temperature from 50°C to 90°C in a humid or aqueous atmosphere until complete hardening is obtained.

14. Biomaterial substantially comprising a microcrystalline hydroxyapatite with a Ca/P atomic ratio from 1.50 to 1.67, obtained by a method according to any of claims 1 to 13, having the following characteristics:
- a compressive strength substantially from 15 to 25 Megapascal,
- a solubility corresponding to a material with a solubility from 10⁻⁹⁴ to 10⁻¹⁰⁰,
- and a specific surface area substantially from 20 to 120 m²/g.

15. A surgical or dental set for carrying out the method according to any of claims 1 to 13, **characterised in that** it includes:
- a measured quantity of solid powdery material including a mixture of tricalcium phosphate and tetracalcium phosphate in a Ca/P atomic ratio substantially from 1.40 to 1.90, said measured quantity being packed in a first closed, sterilised container,
- a measured quantity of aqueous solution containing calcium ions and phosphate ions in a Ca/P atomic ratio substantially from 0.20 to 0.50, said measured quantity being packed in a second closed, sterilised container,
- said measured quantities of solid powdery material and aqueous solution being such that the total liquid/solid weight ratio is from 0.30 to 0.65 and the total Ca/P atomic ratio is from 1.50 to 1.67.

16. A surgical or dental set according to claim 15, wherein the measured quantity of solid, powdery material contains a percentage by weight of sodium, potassium or calcium glycerophosphate from 5% to 15%.

17. A surgical set according to claim 15 or 16, **characterised in that** it includes measured quantities corresponding to one, two or four times the following unit quantities:
- solid powdery material containing from 4.2 to 4.5 g of α tricalcium phosphate and 5.5 to 5.8 g of tetracalcium phosphate, and if appropriate from 1.4 to 1.6 g of sodium, potassium or calcium glycerophosphate,
- aqueous solution containing from 0.18 to 0.22 g of calcium hydroxide and from 1.3 to 1.5 g of phosphoric acid, dissolved in 3.8 and 4.2 g of water.

18. A dental set according to claim 15 or 16, **characterised in that** it comprises measured quantities corresponding to once or twice the following unit quantities:
- solid powdery material containing from 0.42 to 0.45 g of α tricalcium phosphate and from 0.55 to 0.58 g of tetracalcium phosphate, and if appropriate from 0.14 to 0.16 g of sodium, potassium or calcium glycerophosphate,
- aqueous solution containing from 0.02 to 0.025 g of calcium hydroxide and from 0.13 to 0.15 g of phosphoric acid, dissolved in 0.3 and 0.4 g of water.
